(19) Europäisches Patentamt
European Patent Office
Office européen des brevets

(11) Veröffentlichungsnummer: **0 518 088 A2**

# (12) EUROPÄISCHE PATENTANMELDUNG

(21) Anmeldenummer: **92108509.8**

(22) Anmeldetag: **20.05.92**

(51) Int. Cl.[5]: **C12N 9/52**, C12P 21/02, C07K 3/08

(30) Priorität: **23.05.91 DE 4116753**

(43) Veröffentlichungstag der Anmeldung: **16.12.92 Patentblatt 92/51**

(84) Benannte Vertragsstaaten: **BE CH DE DK FR GB IT LI NL**

(71) Anmelder: **HOECHST AKTIENGESELLSCHAFT**
**Postfach 80 03 20**
**W-6230 Frankfurt am Main 80(DE)**

(72) Erfinder: **Müller, Wolfgang, Dr.**
**Im Hopfengarten 11c**
**W-6200 Wiesbaden(DE)**
Erfinder: **Meiwes, Johannes, Dr.**
**Theodor-Fliedner-Str. 39**
**W-6270 Idstein(DE)**

## (54) Verfahren zur Clostripain-katalysierten Verknüpfung von Arg-Pro und Arg-B (B = proteinogene und nicht-proteinogene Aminosäuren) enthaltenden Peptiden.

(57) Für die Verknüpfung von aminoterminal geschütztem Arginin oder von aminoterminal geschützten Peptiden mit carboxyterminalem Arginin (Acyldonor) mit einer Aminosäure oder einem Peptid mit Amindonor-Funktion wird das aus dem Kulturfiltrat von Clostridium histolyticum DSM 627 gewonnene Clostripain eingesetzt. Das Enzym kann durch Alkoholfällung und anschließender Adsorption des aus dem alkoholgefällten Kulturfiltrat gewonnenen Enzyms an Reaktivfarbstoff in hoher Reinheit gewonnen und für die Verknüpfung verwendet werden. Die spezifische Aktivität von Clostripain beträgt mindestens 80 U/mg. Clostripain katalysiert außer der oben erwähnten Verknüpfung von proteinogenen Aminosäuren auch die Verknüpfung einer proteinogenen mit einer nichtproteinogenen Aminosäure.

EP 0 518 088 A2

Enzymatische Peptidsynthesen mit α-Amino-geschützten Argininestern und diversen Amino-ungeschützten, Carboxy-geschützten oder ungeschützten Aminosäurederivaten als Aminodonatoren können prinzipiell mit Metallo-, Serin- oder Thiolproteasen durchgeführt werden. Diese Proteasen sind in der Lage, abhängig von dem Reaktionsgleichgewicht, nicht nur die Spaltung des Peptids bzw. des Proteins in Aminosäuren (= Hydrolyse), sondern auch die Umkehrreaktion zu katalysieren. Vorzugsweise läuft die Peptidsynthese unter kinetischer Kontrolle ab.

Für die Aminolyse von N-geschützten Argininestern eignet sich in vielen Fällen vorzugsweise das preisgünstige Trypsin. Wie jedoch Untersuchungen von Oka (Oka et al. J. Biochem. 82, 1055-1062, 1977) zeigten, gilt dies nicht für den Fall der Verknüpfung mit Prolinderivaten als N-Komponenten.

Mitchell (Science 162, p. 374, 1968) zeigte, daß die Arg-Pro-Bindung in Met-Lys-Bradykininen nicht von Trypsin, jedoch von Clostripain hydrolysiert wird.

Clostripain (EC 3.4.22.8) wurde von Kochalaty (Kochalaty et al. Arch. Biochem. 18,1, 1948) aus Clostridium histolyticum isoliert und von Labouesse und Gros (Bull. Soc. Chim. Biol. 42, 543, 1969), Kula (Biochem. Biophys. Res. Comm. 69, 389, 1976) und Mitchell (J. Biol. Chem. 242, 4683, 1968) charakterisiert.

Die Methode der enzymatischen Peptidverknüpfung von N-geschützten Argininestern mit Prolin-Derivaten bzw. aminoterminal Prolin-enthaltenden Di- und Tripeptiden beschrieben Andersen (Peptides, Proc. 9th APS, p. 355, 1986, Ed. Deber, Hruby) und Fortier (Arch. of Bioch. Biophys. 276, 317, 1990). Andersen und Fortier setzten für ihre Untersuchungen Clostripain ein, welches durch viele hintereinandergeschaltete Schritte hochgereinigt worden war und eine niedrige spezifische Aktivität besaß (z.B. Fortier et al.: 38 U/mg).

Die Herstellung von Clostripain mit einer Aktivität ≧ 80U/mg, welches aus dem Kulturfiltrat von Clostridium histolyticum DSM 627 durch Alkoholfällung des Kulturfiltrats und anschließender Adsorption an Reaktivfarbstoff aufgereinigt wird, sowie seine Verwendung zur enzymatischen Peptidverknüpfung von N-geschützten Argininestern mit Prolin-Derivaten bzw. aminoterminal Prolin-enthaltenden Peptiden wurde noch nicht beschrieben.

Nicht bekannt ist ferner die Clostripain-katalysierte Peptidsynthese mit nichtproteinogenen Aminosäuren als Amindonator statt der Aminosäure Prolin, sowie die Verwendung von Clostripain-Immobilisat für die Verknüpfungsreaktion.

Alle in der Literatur bisher beschriebenen Peptidverknüpfungen mit Clostripain wurden ferner nur im analytischen Maßstab ohne präparative Isolierung der Produkte durchgeführt.

Analytischer Maßstab bedeutet, daß das synthetisierte Peptid durch dünnschichtchromatographische Methoden oder HPLC detektiert werden kann.

Von präparativer Isolierung spricht man bei Isolierung des synthetisierten Peptids durch Extraktionsprozesse zur weiteren Verwendung des Peptids im industriellen Maßstab, d.h. in Gramm- oder Kilogrammengen.

Es ist bis jetzt außerdem kein Verfahren bekannt, mit dem man Arg-Pro oder Arg-B enthaltende Peptidverbindungen mittels Clostripain im industriellen Maßstab herstellen kann.

Es sind zahlreiche Peptidwirkstoffe, die in ihrer Sequenz ein Arg-Pro-Teilfragment aufweisen, bekannt. Sie werden teilweise als Pharmazeutika eingesetzt. So z.B. LHRH (Gly-His-Trp-Ser-Tyr-Gly-Leu-Arg-Pro-Gly-$NH_2$) bzw. LHRH-Analoga (Gonadoliberin-Agonisten, Antagonisten) wie z.B. Buserelin (Gly-His-Trp-Ser-Tyr-D-Ser(tBu)-Leu-Arg-Pro-NHEt) u.a.m. Kürzere Peptide mit der Teilsequenz Arg-Pro haben ebenfalls pharmakologisch interessante Eigenschaften.

Enzymatische Peptidverknüpfungsverfahren zeichnen sich besonders durch das Ausbleiben störender Racemisierung sowie den möglichen Verzicht auf den Seitenketten-Schutz aus. So bietet speziell im Fall des Arginins das enzymatische Verfahren den Vorteil, daß die Guanidino-Gruppe nicht mit teuren, sowie schwer einführ- und abspaltbaren Schutzgruppen (z.B. -Mtr) modifiziert werden muß.

Die meisten industriell produzierten Peptidwirkstoffe werden nach der Methode der klassischer Peptidsynthese in Lösung synthetisiert.

Enzymatische Peptidverknüpfungen können in wäßrigen, wäßrig/organischen oder rein organischen Lösemittelsystemen durchgeführt werden. Verwendet man z.B. wäßriges Methanol als Solvens, ist eine Isolierung der Produkte mit einfachen Aufarbeitungsprozessen nicht möglich, da die noch ausreichend wasserlöslichen Arg-Peptide in der Regel nicht präzipitieren bzw. mit einem organischen Extraktionsmittel wie Essigsäureethylester oder Methylisobutylketon nur ungenügend extrahierbar sind.

Die Verwendung einer Mischphasen-Lösung für die Peptidreaktion, die gleichzeitig Reaktions- und Extraktionsmedium darstellt, ist bisher noch nicht beschrieben.

Es wurde nun gefunden, daß die enzymatische Peptidsynthese zur Darstellung von Peptiden, die Arginin als Acyldonor und proteinogene und nicht-proteinogene Aminosäuren als Amindonoren enthalten,

von Clostripain aus methanolgefälltem Kulturfiltrat von Clostridium histolyticum DSM 627 und von adsorptiv an Reaktivfarbstoff aufgereinigtem Enzym effektiv katalysiert wird.

Die Erfindung betrifft somit:

1. Clostripain mit einer Enzymaktivität von mindestens 80 U/mg erhältlich aus Clostridium histolyticum DSM 627 durch folgendes Herstellverfahren:
   - Fermentation von Clostridium histolyticum DSM 627
   - Herstellung eines Kulturfiltrats
   - Alkoholfällung des Clostripains aus dem Kulturfiltrat
   - Adsorption des alkoholgefällten Clostripains an Reaktivfarbstoff.
2. Verfahren zur Herstellung von Clostripain, das dadurch gekennzeichnet ist, daß Clostripain mit einer Enzymaktivität von mindestens 80 U/mg durch Fermentation von Clostridium histolyticum DSM 627 hergestellt wird.
3. Verwendung des Clostripains zur Verknüpfung von aminoterminal geschütztem Arginin oder von aminoterminal geschützten Peptiden mit carboxyterminalem Arginin (Acyldonor) mit einer Aminosäure oder einem Peptid mit Amindonor-Funktion, welche jeweils eine Carboxylschutzgruppe, ein Carbonsäureamid oder -hydrazid besitzen.
4. Verwendung des Clostripains zur Verknüpfung einer Aminosäure oder eines Peptids mit einer nicht-proteinogenen Aminosäure, welche Amindonor-Funktion hat oder mit einem Peptid, welches eine derartige Aminosäure besitzt.

Im folgenden wird die Erfindung in ihren bevorzugten Ausführungsformen beschrieben. Ferner wird die Erfindung durch den Inhalt der Ansprüche bestimmt.

Bei dem erfindungsgemäßen Verfahren katalysiert Clostripain die Verknüpfung von Aminosäuren oder Peptiden, die als Acyldonor Arginin aufweisen.

Das erfindungsgemäße Clostripain wird wie folgt gewonnen:

- Fermentation von Clostridium histolyticum DSM 627
- Herstellung eines Kulturfiltrats durch Zentrifugaton und Sterilfiltration
- Alkoholfällung des Clostripains aus dem Kulturfiltrat, insbesondere Methanolfällung und
- Adsorption des alkoholgefällten Clostripains an Reaktivfarbstoff, insbesondere an Reactive Red 120

Das erfindungsgemäße Clostripain kann außerdem wie folgt gewonnen werden (s. Schema 1):

- aus sterilfiltriertem Kulturfiltrat von Clostridium histolyticum DSM627 welches vorzugsweise 50-fach aufkonzentriert wurde oder
- aus ultrafiltriertem Kulturfiltrat von Clostridium histolyticum DSM627 oder
- aus Kulturfiltrat von Clostridium histolyticum DSM627 nach der Alkoholfällung, insbesondere nach der Methanolfällung oder
- durch Ammoniumsulfatfällung des ultrafiltrierten Kulturfiltrats von Clostridium histolyticum DSM627 oder
- durch Adsorption von Clostripain, gewonnen aus alkohol-, insbesondere methanolgefälltem Kulturfiltrat von Clostridium histolyticum DSM627 und Adsorption an Reaktivfarbstoff, insbesondere an Reactive Red 120, oder
- durch hydrophobe Chromatographie der ammoniumsulfat- oder alkoholgefällten Kulturbrühe insbesondere an Aminobutyl- oder Hexylagarose.

Die Gewinnung von Clostripain ist ebenfalls durch eine andere Kombination der in Schema 1 angegebenen Aufreinigungsschritte möglich.

Schema 1: Gewinnung von Clostripain

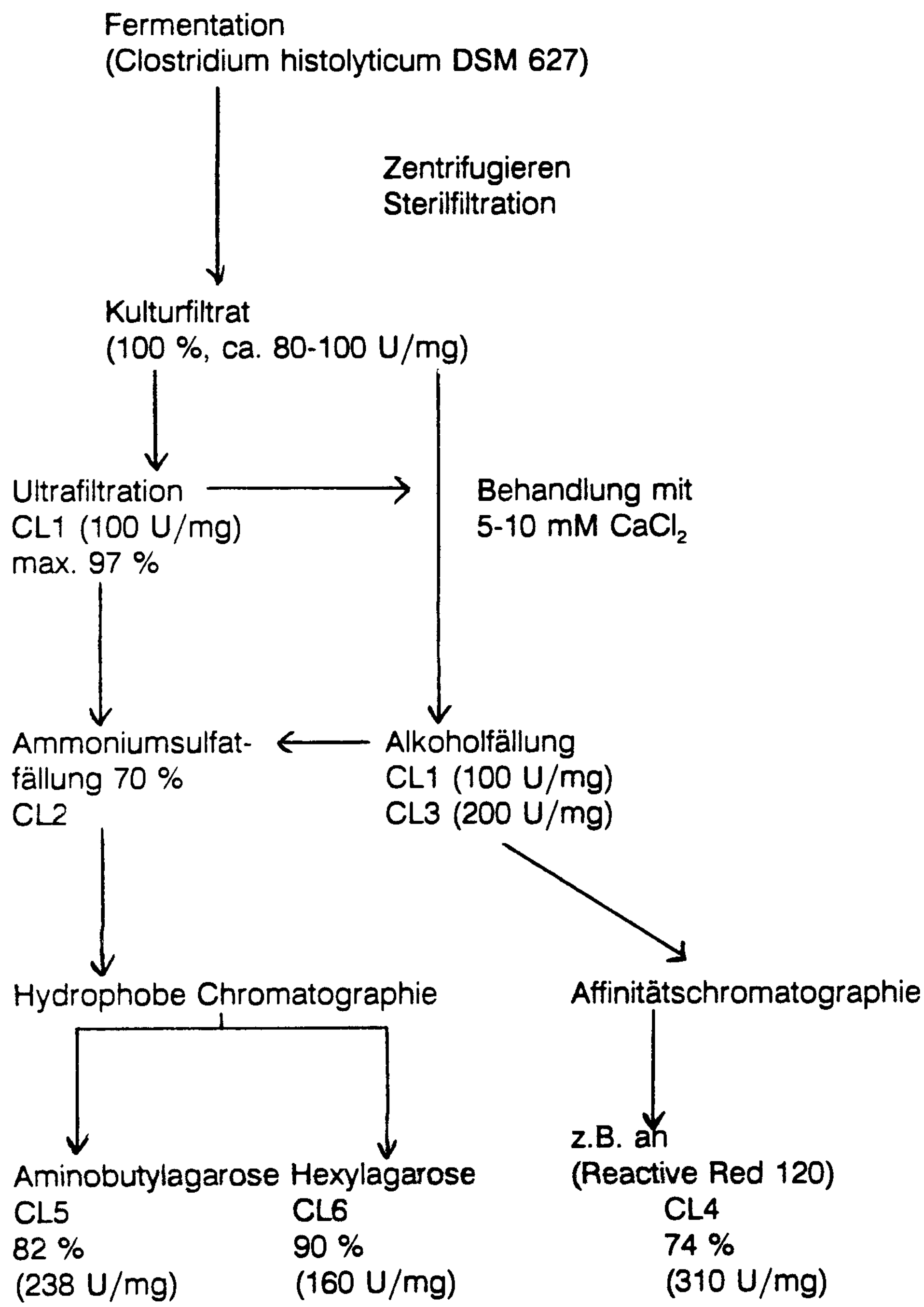


Für die Gewinnung des Clostripain wird der Stamm DSM 627 für 12-28 Stunden mit dem optimierten Nährmedium NL 1581 oder dem Proteose-Pepton-Medium fermentiert. Die Temperatur der Vorkultur beträgt 33-39 ° C, vorzugsweise 37 ° C, die der Hauptkultur 31-36 ° C, vorzugsweise 33 ° C.

Der Stamm DSM627 ist bei der Deutschen Gesellschaft für Mikroorganismen und Zellkulturen, Mascheroder Weg 1B, 3300 Braunschweig, frei verfügbar.

Nährmedium NL 1581:
(Die Angaben beziehen sich auf 1l Nährmedium)

| | |
|---|---|
| - 30 g | Fleischextrakt |
| - 30 g | Casein-Pepton |
| - 5 g | Hefeextrakt |
| - 1,74 g | $K_2HPO_4$ |
| - 0,5 g | Cystein |
| pH 7,4 | |

Proteose-Pepton-Medium:
(Die Angaben beziehen sich auf 1l Medium)

| | |
|---|---|
| - 50 g | Proteose Pepton |
| - 1,74 g | $K_2HPO_4$ |
| - 0,5g | Cystein |
| pH 7,2 | |

Die Fermentation mit dem optimierten Nährmedium NL 1581 oder dem Proteose-Pepton-Medium ergibt nach Zentrifugation (10.000 g für 10 Min.) und Sterilfiltration (Porengröße der Membran 0,2-0,4 $\mu$m, vorzugsweise 0,3 $\mu$m, wie z.B. ®Omega 0,3 $\mu$m-Filter der Firma Filtron) das Kulturfiltrat mit bis zu 50000 U/L (entspricht 60-120, insbesondere 80-100 U/mg Clostripain), welches mittels Ultrafiltration ohne Proteinverlust aufkonzentriert wird. Diese Enzymqualität wird als CL1 bezeichnet.

Die Ultrafiltration dient der Konzentrierung der Kulturbrühe.

Als Ultrafiltrationsmembran sind alle Membranen mit einer Porengröße 5-50, vorzugsweise 10 KD (KD = Kilodalton) geeignet (z.B. Omega 10 K v.d. Firma Filtron). Größere Chargen (> 5l - 1m$^3$) werden jedoch mittels Tangentialfluß (Crossflow)-Kassettensystem aufkonzentriert.

Bereits mit dem aus der Ultrafiltration gewonnenen Clostripain können Verknüpfungen durchgeführt werden. Das Enzym hat eine Aktivität von 80-100, insbesondere 90 U/mg.

Eine alternative Aufarbeitung über eine Ammoniumsulfat-Fällung (40 bzw. 70 % $(NH_4)_2SO_4$) analog Labouesse (Bull. Soc. Chim. Biol. 42, 543-68, 1960) führte zur Qualität CL2 in 32 % Ausbeute, während die Alkohol-, insbesondere die Methanol-Fällung analog Ogle (Arch. Biochem. Biophys 42, 327-36, 1953) CL3 in rund 70 % Ausbeute Enzym mit der spezifischen Aktivität bis zu 250 U/mg-Protein liefert.

Das durch die Ammoniumsulfatfällung gewonnene Clostripain kann mittels hydrophober Chromatographie, insbesondere an Aminobutyl- oder Hexylagarose weiter aufgereinigt werden. Das Enzym besitzt dann eine Qualität von 150-250, insbesondere 150-170 (Hexylagarose) bzw. 220-250 U/mg (Aminobutylagarose).

Vor der Methanolfällung muß zur Stabilisierung des Enzyms unbedingt der $Ca^{2+}$ Ionengehalt überprüft werden, andernfalls muß $CaCl_2$ oder Calciumacetat zugefügt werden (Sollwert: 5-10 mM, 60 °C, 15 Min.). Dabei geht man so vor, daß man soviel $CaCl_2$ oder Calciumacetat zur Kulturbrühe gibt, bis die Endkonzentration der Kulturbrühe 5-10 mM erreicht.

Die Methanolfällung gestattet eine kostengünstige und auch im Gramm-Maßstab durchführbare Enzymgewinnung der Qualität CL3.

Das mittels Alkoholfällung gewonnene Clostripain kann durch Adsorption an Reaktivfarbstoff, insbesondere Reactive Red 120 [Sigma], und anschließender Elution mittels Salzen, vorzugsweise Na- oder KCl (0,4 M) nochmals aufgereinigt werden. Diese Vorgehensweise erlaubt die Gewinnung von hochreinem Clostripain in einem nur zweistufigen Vorgang (1. Methanolfällung des Kulturfiltrats und 2. Adsorption des im ersten Schritt gewonnenen Enzyms an Reaktivfarbstoff). Darüberhinaus werden hohe Ausbeuten erzielt. Die Enzymaktivität beträgt 250-350, insbesondere 280-330 U/mg.

Clostripain kann für die Verknüpfungsreaktion in immobilisierter und nichtimmobilisierter Form eingesetzt werden.

Als Trägermaterialien werden vorzugsweise adsorptiv wirkende Träger, wie z.B. Kieselgel oder CNBr-Sepharose eingesetzt. Die Träger sind handelsüblich erhältlich.

Zur Immobilisierung des Enzyms wird dieses mit dem Träger vermischt. Die Dauer der Kopplungsreaktion, das Reaktionsmilieu und das Mischungsverhältnis von Enzym und Träger ist von der Art des gewählten Trägers abhängig und kann für jeden Einzelfall durch Vorversuche vom Fachmann leicht bestimmt werden.

Für die Peptidkopplung muß eine Aminokomponente ( = Amindonor) und eine Carboxykomponente ( =

Acyldonor) vorhanden sein.

Als Carboxykomponente werden N-geschützte Argininalkylester bzw. Peptidfragmente, welche am Carboxyterminus eine Argininalkylestergruppe aufweisen, vorzugsweise jedoch Z-Arg-OMe, Z-Leu-Arg-OMe, Z-Gly-Pro-Arg-OMe, Boc-D-Arg-Arg-OMe, verwendet.

Als Aminokomponente werden Aminosäurederivate bzw. Peptidfragmente, welche als Aminoterminus eine proteinogene Aminosäure, mit Ausnahme der Aminosäuren, Asn, Gln oder Thr aufweisen, Prolinester und -amide, vorzugsweise Pro-$NH_2$, Pro-NHEt, Pro-Gly-$NH_2$, Pro-Ala-$NH_2$, Pro-AzaGly-$NH_2$, D-Aminosäuren oder nichtproteinogene Aminosäuren wie z.B. Halogen substituierte Phenylalanine, Furyl-, Thienylalanine, Phenylglycin, Homophenylalanin, 2-Aminonorbornan-2-carbonsäure oder phosphorhaltige Aminosäuren verwendet.

Die zu koppelnden Aminosäuren bzw. Peptide müssen vor der Kopplungsreaktion nach dem Fachmann bekannten Methoden amino-(Carboxykomponente) bzw. carboxygeschützt (Aminokomponente) werden.

Hierfür eignen sich die Aminoschutzgruppen: Z, Boc, Ac, For, Bz und die Carboxylschutzgruppen OR (mit R = $C_1$-$C_5$, Bzl, $tBU_x$) $NH_2$ und NHR' (R' = $C_1$-$C_5$, NH-$CONH_2$). Statt der Carboxylschutzgruppe kann auch ein Carbonsäureamid oder -hydrazid verwendet werden.

Die Reaktionsbedingungen für die Kopplungsreaktion sind abhängig von den zu koppelnden Aminosäuren bzw. Peptiden und sind in den Beispielen aufgeführt. Das Nucleophil wird in 2-10 fachem Überschuß zugesetzt. Prolinamid oder prolinhaltige Nucleophile mit N-terminalem Prolin werden vorzugsweise in 2-6 fachem Überschuß im Vergleich zum Acyldonor eingesetzt.

Die Peptidkopplung kann bei diesem geringen Nucleophilüberschuß nur erfolgreich durchgeführt werden, wenn

a) die Methanolkonzentration und gleichzeitig

b) die absolute Konzentration der Reaktanden erhöht wird.

Die Methanolkonzentration liegt bei Verwendung des 2-6 fachen Überschusses im Bereich von 40-90%. Die Konzentration der Reaktanden wird bei 2-6 fachem Überschuß im Vergleich zum 10fachen Überschuß bis um den Faktor 3 erhöht. Da trotz des hohen Methanolanteils die Löslichkeitsgrenze der Argininderivate überschritten wird, müssen die Aminosäuren in der Hitze vorgelöst und erst nach dem Abkühlen der Lösung das Enzym zugegeben werden.

Außer Methanol, welches vorzugsweise zur Peptidkopplung unter der Bedingung des geringen Nucleophilüberschusses eingesetzt wird, können außerdem noch verwendet werden: THF (Tetrahydrofuran), Dioxan, Dimethoxyethan, Ethylenglycol, Diethylenglycoldiethylether oder Ethanol.

Die für Methanol angegebenen Reaktionsbedingungen gelten ebenso für diese Lösemittel.

Die Verwendung des Lösemittels Butanol erfordert einen 10fachen Nucleophilüberschuß.

Die Peptidkopplung kann auch in einem Mischphasen-System durchgeführt werden, welches gleichzeitig Reaktions- und Extraktionsmedium darstellt. Dieses Mischphasen-System besteht vorzugsweise aus Butanol und wäßriger NaCl-Lösung.

Die Umsetzung erfolgt im kontinuierlichen oder diskontinuierlichen Verfahren.

Wie oben beschrieben, findet die Verknüpfung vorzugsweise in einem Mischphasen-System statt, welches gleichzeitig Reaktions- und Extraktionsmedium ist. Durch Extraktion erfolgt Überführung des Peptids in die Butanolphase, welche nach der Phasenseparation abgetrennt wird. Ein mehrmaliges Ausschütteln der Kochsalzphase mit Butanol erhöht die Peptidausbeute.

## BEISPIELE

### I. Immobilisierung von Clostripain:

Clostripain kann ionogen an DEAE-Sephadex® A 50 (Pharmacia) gebunden werden. Die kovalente Immobilisierung ist jedoch für Clostripain vorteilhafter.

So werden für die in Tabelle 1 aufgeführten Materialien z.T. sehr hohe Bindungsausbeuten erreicht.

Vor der Immobilisierung kann Clostripain mit dem Reduktionsmittel Dithiothreitol (DTT) aktiviert werden. Es ist jedoch auch möglich, das Enzym in nicht-aktivierter Form zu verwenden. Die höchste Aktivität pro ml Träger wird mit Bromcyan-aktivierter ®Sepharose 4B erreicht. Dieses Immobilisat hat jedoch nur eine mäßige Langzeitstabilität.

Robustere Träger wie das VA-Epoxy-Immobilisat oder Glutardialdehyd/Kieselgel weisen eine wesentlich geringere Belegungsdichte auf.

Tabelle 1: Immobilisierung von Clostripain an verschiedene Träger

| Trägermaterial | DTT-Voraktivierung | Bindungsausbeute % | Aktivität U/ml | Langzeitstabilität |
|---|---|---|---|---|
| DEAE-Sepharose A 50 | nein | 32 | 13 | - - |
| " " | ja | 24 | 103 | |
| Kieselgel " | ja | 95 | 59 | + + |
| " | nein | 95 | 46 | |
| Eupergit C | nein | 90 | 432 | + |
| " | ja | 99 | 166 | |
| VA-Epoxy | ja | 99 | 66 | + |
| " | nein | 92 | 16 | |
| Tresyl Sepharose 4B | ja | 13 | 17 | n.b. |
| " | nein | 36 | 19 | |
| Bromcyan-Sepharose 4B | ja | 34 | 45 | - |
| " | nein | 92 | 500 | - |

DEAE-Sepharose A 50-Clostripain:

Die Immobilisierung erfolgte analog H. Woodward, Imm. Cells and Enzymes, IRL Press, Oxford 1985.

Kieselgel-Clostripain:

Die Immobilisierung erfolgte analog EP 3 438 189.

50 ml Kieselgel (Grace) wird mit 100 ml Wasser und 3 g Aminopropylethoxysilan versetzt, 2 h bei 65 ° C gerührt, heiß filtriert und 24 h bei 90 ° C getrocknet.

Danach wird ein Volumenteil aminierter Träger mit 2 Volumenteile 0,25 M Kaliumphosphatpuffer pH 8, mit 2,5 % Glutardialdehyd versetzt und 2 Std. leicht geschüttelt.

Nach Absaugen des Trägers und mehrmaligem Waschen, beträgt die Ausbeute an fixiertem Enzym 90 - 95 %. Die Belegungsdichte liegt bei 45 - 70 U/ml feuchten Träger.

Das Kieselgel-Immobilisat ist aufgrund seines günstigen Preises besonders für präparative Zwecke geeignet.

Eupergit C (Röhm):

Immobilisierung in 0,5 M Kaliumphosphatpuffer pH 7,5/4 ° C/48 h.

VA-Epoxy (Riedel de Haën)

Immobilisierung in 1M Kaliumphosphatpuffer pH 7,5/20 ° C/24 h.

Tresyl Sepharose 4B (Pharmacia):

Durchführung analog Nilsson, Mosbach Biotech. Bioeng. 26, 1146-54, 1984.

Bromcyan Sepharose B (Pharmacia):

Durchführung analog Cobbs, Biotechnol. Techn. 5, 5-10, 1990.

2. Test unterschiedlicher Aufreinigungsfraktionen des Clostripains auf die Peptidsyntheseaktivität bzw. konkurrierende Esterhydrolyse während der Reaktion von Z-Arg-OMe mit $Pro\text{-}NH_2$ in 30 %iger Methanollösung (Z-Arg-OMe:$Pro\text{-}NH_2$ = 1:10)

Mit diesen Untersuchungen sollte geklärt werden, inwieweit das Enzym aufgereinigt werden muß, um eine Arg-Pro-Knüpfung mit hoher Ausbeute und möglichst geringer Verseifung des Argininesters zu erhalten.

Es wurde die Bildung von Z-Arg-Pro-$NH_2$ und Z-Arg-OH in Abhängigkeit der eingesetzten Enzymqualität nach 10 und 30 Min. per HPLC bestimmt.

Die Produktbildung (Z-Arg-Pro-$NH_2$) im Verhältnis zur Konkurrenzhydrolyse des Z-Arg-OMe zu Z-Arg-OH ist ein Hinweis auf die Güte des Reaktionsverlaufs der Peptidsynthese.

Z-Arg-OH entsteht als Nebenprodukt durch die Konkurrenzhydrolyse von Z-Arg-OMe. Die Menge dieses Nebenproduktes ist in der Regel um so höher, je schlechter die Donoreigenschaften des Nucelophils sind oder Verunreinigungen des katalysierenden Enzyms sich störend auf den Reaktionsverlauf auswirken. Z-Arg-OH ist darüberhinaus sehr schlecht von dem entstandenen Peptid abtrennbar. Die Peptidsynthese ist um so erfolgreicher abgelaufen, je weniger Z-Arg-OH entsteht.

Nach 10 bzw. 30 Min. Reaktionszeit entstanden folgende Mengen von Z-Arg-OH bzw. Z-Arg-Pro-$NH_2$:
Bei Verwendung des Clostripains aus

| | Z - Arg - OH | | Z-Arg - Pro - $NH_2$ | | |
|---|---|---|---|---|---|
| | 10 Min | 30 Min | 10 Min | 30 Min | % |
| - ultrafiltriertem Kultufiltrat (CL1): | 2 | 2,2 | 53 | 85 | % |
| - der Methanolfällung (CL3): | 2,5 | 3,9 | 67 | 91 | % |
| - der 70%igen Ammoniumsulfat-fällung (CL2): | 0 | 0,7 | 64 | 88 | % |
| - der 40%igen Ammoniumsulfat-fällung (CL2): | 1,8 | 2,4 | 78 | 97 | % |
| - der Aminobutylagarose-Aufreinigung (CL5): | 1,8 | 2,5 | 61 | 88 | % |
| - und des Clostripainlyophilisats nach der Reactive Red-Behandlung(CL4): | 2,7 | 1,8 | 60 | 90 | % |

Die in Prozenten angegebene Menge Z-Arg-OH bzw. Z-Arg-Pro-$NH_2$ ist bezogen auf die Gesamtmenge von Z-Arg-Pro-$NH_2$, Z-Arg-OH und Z-Arg-OMe.

III. Peptidsynthesen mit Clostripain: Peptidverknüpfung:
A). Peptidverknüpfung ...Arg-Pro-...

Beispiel 1a:

Synthese von Z-Arg-Pro-$NH_2$ mit löslichem Clostripain der Qualität CL1: 358,8 mg (1 mmol) Z-Arg-OMe x HCl sowie 1,5 g (10 mmol) Pro-$NH_2$ xHCl werden in 3 ml 30 % Methanol gelöst, der pH-Wert auf 7,8 eingestellt und bei 40 ° C mit 26 U Clostripain (CL1) inkubiert. 1 U Clostripain ist definiert als die Menge Enzym, die 1 mol N-Benzoyl-L-Argininethylester/min bei 25 ° C, pH 7,6 in Anwesenheit von 2,5 mM Dithiothreitol hydrolysiert.

Während der Reaktion wird der pH-Wert konstant gehalten. Nach 90 Min. erreicht man laut HPLC 94 % Peptid. Zur Isolierung wird Methanol entfernt, die wäßrige Lösung mit Kochsalz gesättigt und mehrmals mit n-Butanol ausgeschüttelt. Die eingeengte Butanolphase enthält analysenreines Peptid in 85 - 90 % Ausbeute.

Beispiel 1b:

Der Ansatz erfolgt wie in Beispiel 1a, jedoch wird die angegebene Menge Z-Arg-OMe zusammen mit 450 mg (2,5 eq.) H-Pro-$NH_2$*HCl in der Hitze in 1 ml 50 %igem MeOH gelöst und unmittelbar nach dem Abkühlen auf 40 ° C mit dem Enzym versetzt. Nach einer Reaktionszeit von 1 h hat man laut HPLC einen Umsatz von 93 % erreicht. Aufarbeitung und Produktisolierung erfolgen wie unter 1a beschrieben.

Beispiel 2:
Synthese von Z-Arg-Pro-$NH_2$ mit immobilisiertem CL1 im Methanol/Wasser-System

Ansatz wie in Beispiel 1, jedoch Zugabe von 100 U Clostripain-Kieselgel-Immobilisat. Der Ansatz wird unter pH-statischen Bedingungen langsam gerührt. Nach 40 min liegen 95 % Peptid vor, der Katalysator kann abgetrennt und mit Puffer gewaschen werden.

Beispiel 3:
Synthese von Z-Arg-Pro-NH2 mit immobilisiertem Clostripain in NaCl-Lösung (6.1M)/n-Butanol = (50:50 im Batch-Betrieb).

1 mmol Z-Arg-OMe x HCl und 10 mmol Pro-$NH_2$ x HCl werden in 1,5 ml 6.1M NaCl-Lösung und 1,5 ml n-Butanol bei pH 7,8 und 40 ° C gelöst. Es werden 48 U Kieselgel-Immobilisat zugegeben. Nach 40 Min. sind 89 % Peptid gebildet. Nach der Phasenseparation wird die Butanolphase abgetrennt und die Kochsalzphase noch dreimal mit n-Butanol ausgeschüttelt. Man isoliert 78 % Z-Arg-Pro-$NH_2$.

Beispiel 4:
Synthese von Z-Arg-Pro-Gly-$NH_2$ mit Säulenbettreaktor/NaCl-Butanol:

7,18 g (20 mMol) Z-Arg-OMe x HCl und 41,5 g (200 mmol) H-Pro-Gly-$NH_2$ x HCl werden in 30 ml gesättigter NaCl-Lösung und 30 ml Butanol gelöst, mit 16,5 ml 10 N NaOH wird der pH auf 7,8 eingestellt, die Reaktionstemperatur beträgt 40 ° C.

Mit einer Umpumpgeschwindigkeit von ca. 14 ml/min wird der Ansatz über eine 25 ml-Clostripain-Immobilisat-Säule geführt.

Nach 80 Min wird die Reaktion abgebrochen und es werden die Phasen separiert. Das Tripeptid wurde mit 86 %iger Ausbeute aus der Butanolphase isoliert. Die verbleibende Solephase kann nun wieder mit den abreagierten Äquivalenten nachgespeist werden und erneut über die Enzymsäule gepumpt werden.

Nach 4-5 maliger Verwendung der Solephase konnte das Diketopiperazinderivat cyclo-(Pro-Gly) beobachtet werden, was insgesamt zu einer Verzögerung der Reaktionsgeschwindigkeit führte.

Beispiel 5:
Weitere Arg-Pro-Verknüpfungen

Es wurde die Synthese von Clostripain-katalysierten Peptidsynthesen mit Z-Arg-OMe als Acyldonor (also Arg in P1-Position) und Prolin (als aminoterminale Aminosäure des Nucelophils) in P1- und besetzter P2'-, P3'-Position durchgeführt.

| z.B.: | P2 | P1 | P1' | P2' | P3' |
|---|---|---|---|---|---|

Z-----Arg-----Pro----Ala----$NH_2$

Tabelle 2:

| Nucleophil | Peptid | (Ausbeute-Bereich %) |
|---|---|---|
| Pro-NHEt | Z-Arg-Pro-NHEt | 80 - 96 |
| Pro-Gly-$NH_2$xHCl | Z-Arg-Pro-Gly-$NH_2$ | 70 - 90 |
| Pro-Azagly-$NH_2$xHCl | Z-Arg-Pro-AzaGly-$NH_2$ | 80 - 90 |
| Pro-Ala-$NH_2$xHCl | Z-Arg-Pro-Ala-$NH_2$ | 85 - 95 |

a) Z-Arg-Pro-NHEt:
Reaktionsansatz:
Ester:Amin = 1:10 in 10 % MeOH, pH 7,8; 0,25 ml CL3 (230 U/ml) pro 1mmol Ester; Reaktionszeit: 30 Min.;
HPLC-Ausbeute: 80 %.
b) Z-Arg-Pro-NHEt:
Reaktionsansatz:
Ester:Amin = 1:10 in NaCl/BuOH = 1:1, pH 8,3; 0,5 ml CL3 (230 U/ml) pro 1mmol Ester; Reaktionszeit 50 Min.;
HPLC-Ausbeute: 96%
c) Z-Arg-Pro-Gly-$NH_2$:
Reaktionsansatz:
Ester:Amin = 1:10 in NaCL-BuOH (1:1), 2 ml CL3-Kieselgel pro 1mmol Ester;
Reaktionszeit 1 Std.;
HPLC-Ausbeute: >95 %.
Die Butanol-Phase enthält Peptid mit 94 % Reinheit.
d) Z-Arg-Pro-Ala-$NH_2$:
Reaktionsansatz:
Ester:Amin = 1:10, in 10 % MeOH; 0,25 ml (CL3-Lösung (230 U/ml) pro 0,05 mmol Ester. Nach 45 Min. ist kein Ester mehr nachweisbar.
Laut HPLC entstand ca. 94 % Peptid, 5 % Z-Arg-OH.
e) Z-Arg-Pro-OCAM:
CAM =

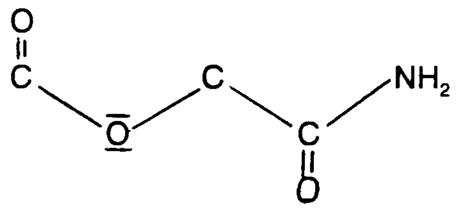

Reaktionsansatz:
Ester:Amin = 1:10 in 10% MeOH,
0,15 ml CL3-Lösung (400 U/ml) pro 1 mmol Ester; Reaktionszeit: 2 h.
HPLC-Ausbeute: 30 %
f) Z-Arg-Pro-$N_2H_3$
Reaktionsansatz:
Ester:Amin = 1:10 in 10 % MeOH;
0,1 ml CL3-Lösung (600 U/ml) pro 1 mmol Ester; Reaktionszeit : 3/4 h
HPLC-Ausbeute: 56 %
g) Z-Arg-Hyp-$NH_2$
Reaktionsansatz:
Ester:Amin = 1:10 in 10 % MeOH;
0,15 ml CL3-Lösung (660 U/ml) pro 1 mmol Ester; Reaktionszeit: 1,5 h
HPLC-Ausbeute: 81 %
B) Peptidverknüpfung von nichtproteinogenen Aminosäuren als Amindonor

1 mmol Z-Arg-OMe x HCl und 1-2 mmol Nucleophil werden in 2-4 ml 10 %igem wäßrigem Methanol gelöst und mit 5N NaOH auf pH 7,8 - 8 eingestellt.
Es folgt dann die Zugabe von 0,25 - 0,5 ml CL3-Enzymlösung (400 - 500 U/ml). Das Enzym wurde zuvor mindestens 1 Std. mit Aktivierungspuffer behandelt (2,5 mM $CaCL_2$, 10 mM DTT). Die Reaktionslösung wird unter pH-stat. Bedingungen gefahren (Titration mit 1N NaOH).
Einige Produkte fallen direkt aus der Reaktionslösung aus (z.B. Z-Arg-4F-Phe-$NH_2$) und werden durch Filtration/Nachwaschen mit 5 % Methanol/dest. Wasser isoliert.

Nach 0,75 - 2 Std. ist laut HPLC kein Z-Arg-OMe zu erkennen. Es entstand neben dem Peptid 2 - 50 % Z-Arg-OH durch Konkurrenzhydrolyse des eingesetzten Esters.

Man stoppt die Umsetzung durch Zugabe von einigen Tropfen $HClO_4$ (pH ca. 2-3).

Die Lösung wird zur Entfernung des Methanols ca. auf 1/3 des Volumens eingeengt, mit NaCl gesättigt und dreimal mit Butanol extrahiert. Häufig ist das Peptid noch mit wenigen Prozenten an Z-Arg-OH verunreinigt. (Die präparativen Ausbeuten liegen wegen unvollständiger Extraktion häufig erheblich niedriger als die HPLC-Angaben.)

Tabelle 2

| Nucleophil (Formel) | Peptid | HPLC-,Präp.-(Ausbeute - %) |
|---|---|---|
| 4F-Phe-$NH_2$ | Z-Arg-4F-Phe-$NH_2$ | 97,73 |
| 4F-Phe-OMe | Z-Arg-4F-Phe-OMe | 91,70 |
| DL-4F-Phe-OProp | Z-Arg-D,L-4F-Phe-OProp | 90,69 |
| 4F-Phe-4F-Phe-$NH_2$ | Z-Arg-4F-Phe-4F-Phe-$NH_2$ | 85,80 |
| 4Cl-Phe-OProp | Z-Arg-4Cl-Phe-OProp | >95,76 |
| Nal-OProp 1 | Z-Arg-Nal-OProp | >95,88 |
| DL-Phg-OMe 2 | Z-Arg-D,L-Phg-OMe | >95,78 |
| Phgly-OtBu | Z-Arg-Phgly-OtBu | 90,70 |
| HPhe-OEt 3 | Z-Arg-HPhe-OEt | 85,72 |
| DL-DMPO-Ala-OProp 4 | Z-Arg-D,L-DMPO-Ala-OProp | 40,10 |
| Thiaz-Cys-OProp 5 | Z-Arg-Thiaz-Cys-OProp | 70,50 |
| PTC-Phe-$NH_2$ 6 | Z-Arg-PTC-Phe-$NH_2$ | >95,63 |
| Fur-Ala-OMe 7 | Z-Arg-Fur-Ala-OMe | 75,45 |
| Thi-OMe 8 | Z-Arg-Thi-OMe | 73,59 |
| H-Sar(*)-$NH_2$ | Z-Arg-Sar-$NH_2$ | 66 |
| H-Gly-$N_2H_4$ | Z-Arg-Gly-$N_2H_4$ | 92 |
| H-Leu-$N_2H_4$ | Z-Arg-Leu-$N_2H_4$ | 66 |

(*)Sar = N-Methylglycin
(Aminosäurehydrazide müssen eher als nicht proteinogene Aminosäuren als z.B. geschützte Derivate proteinogener Aminosäuren aufgefaßt werden, denn im Gegensatz zu diesen sind Carbonsäurehydrazide der Peptide bzw. Aminosäuren nicht in diese rückspaltbar).
Sar = N-Methylglycin

| Formel: | | Aminosäure-Stammverbindung: |
|---|---|---|
| 1 | 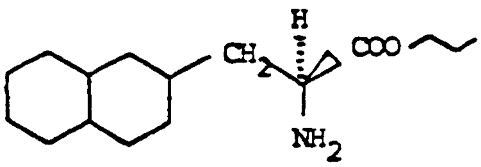 | L-3-(2-Naphthyl)-alanin |
| 2 | 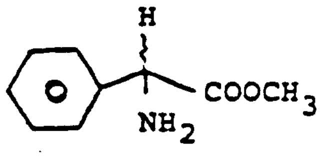 | DL-2-Phenyl-Glycin |
| 3 | 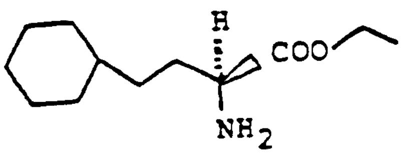 | L-Homophenylalanin |
| 4 | 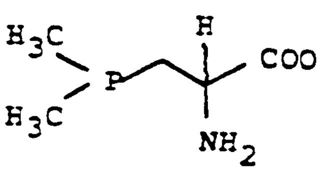 | DL-3-(Dimethylphosphinyl)-Alanin |
| 5 | 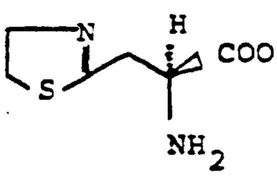 | L-(2-Thiazolinyl)-Cystein |

6 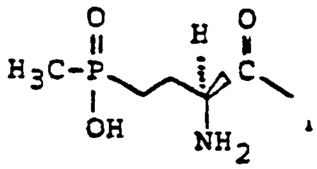 L,L-Phosphinotricinyl-Phenylalanin

7 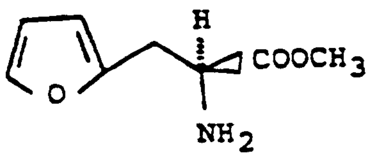 L-3-(2-Furyl)-Alanin

8 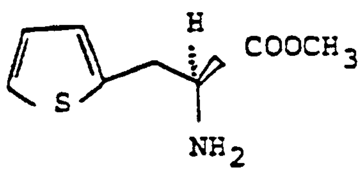 L-3-(2-Thienyl)-Alanin

3. Zweiphasige Verfahren mit Immobilisat im Rührreaktor mit Akkumulierung des Produkts

Es wurden vier aufeinanderfolgende Batchläufe (je 1 mmol) mit Kieselgel/CL3 in einer NaCL/Butanol-Lösung (50 %iges Butanol) durchgeführt.

In diesem System ist die Umsetzung langsamer, die Ausbeute an Peptid niedriger und der prozentuale Anteil an gebildeter Säure höher als in einem vergleichbaren Einphasensystem. Ferner sinkt die Aktivität des Katalysators schneller als beim einphasigen Methanol/Wasser-System.

Nach der Aktivierung vor dem 4. Lauf zeigte das Immobilisat nur noch 50 % seiner Anfangsaktivität.

Beispiel 6:
Z-Arg-Pro-AzaGly-$NH_2$

| Cyclus Nr. | Ester mmol | Amin mmol | 10% MeOH ml | CL3-Lsg* ml | Reakt.zeit min | Ausbeute HPLC |
|---|---|---|---|---|---|---|
| 1 | 5 | 50 | 15 | 3 | 20 | 78 |
| 2 | +5 | +5 | 15 | - | 45 | 81 |
| 3 | +5 | +5 | 20 | 2,5 | 40 | 80 |
| 4 | +5 | +5 | 25 | - | 30 | 79 |
| 5 | +5 | +5 | 15 | 3 | 20 | 76 |
| 6 | +5 | +5 | 20 | 1 | 30 | 84 |

* CL3: 80 U/ml

Nach 6 Cyclen wird abgebrochen und mit n-Butanol extrahiert.

4. Zweiphasige Verfahren mit Immobilisat im Säulenreaktor mit Akkumulierung des Produkts

Wie Vorversuche zeigten, lassen sich die in der Butanol/NaCl-Lösung gelösten Edukte problemlos ohne Phasenseparation über ein Katalysatorbett im Kreislauf pumpen.

Daraufhin wurden je 2 Batchläufe (a 15 mmol) mit einer Kieselgel- bzw. einer Eupergitsäule durchgeführt.

Die Reaktionsgeschwindigkeit ist etwas verlangsamt, die Ausbeute etwas geringer. Im zweiten Batchlauf zeigt die Säule einen deutlichen Aktivitätsschwund. Ferner reagiert der Acyldonor nicht quantitativ ab.

Dennoch lassen sich präparative Mengen mit dieser Methode bequem darstellen, denn ein wichtiger Vorteil des Zweiphasensystems ist darin zu sehen, daß nach der Phasenseparation aus der Butanolphase direkt das Produkt ausgeschleußt werden kann.

Beispiel 7:
Z-Arg-Pro-Gly-$NH_2$:

Es wurden 3 Cyclen gefahren, wobei nur für den ersten Ansatz ein 10-facher Nucleophil-Überschuß gewählt wurde. Für die folgenden Cyclen wurden nur die wegreagierten Äquivalente ersetzt, sowie die Reaktionsgeschwindigkeit durch zusätzliche Enzymzugabe korrigiert.

| Cyclus Nr. | Ester mmol | Amin mmol | BuOH ml | NaCl ml | CL3-Lsg. ml | Reakt.zeit min | Ausbeute HPCl% |
|---|---|---|---|---|---|---|---|
| 1 | 5 | 50 | 12,5 | 12,5 | 0,25 | 60 | >95 |

Nach dem 1. Cyclus wird die Butanolphase abgetrennt, die NaCL-Phase mit den Äquivalenten nachgespeist ->Cyclus 2

| | | | | | | | |
|---|---|---|---|---|---|---|---|
| 2 | +5 | +5 | +12,5 | - | 1,0 | 120 | >95 |

Nach dem 2.Cyclus wird die Butanolphase abgetrennt, die NaCl-Phase mit den Äquivalenten nachgespeist -> 3

| | | | | | | | |
|---|---|---|---|---|---|---|---|
| 3 | +5 | +5 | +12,5 | - | 0,7 | 75 | >90 |

Die vereinigten Butanolphasen werden getrocknet und eingeengt. Nach DC, HPLC sind noch 2-5 % Z-Arg-OH enthalten, die durch mehrmaliges Umkristallisieren entfernt werden können.
Präp. Gesamtausbeute 72 % (Ca. 95 % Reinheit).

Je mehr Cyclen durchgeführt werden, umso rascher sinkt die Ausbeute. Mit zunehmender Reaktionsdauer entsteht bei diesen Reaktionsbedingungen relevante, geringe Mengen des Diketopiperazins: cyclo-(Pro-Gly), welches erstens die effektive Aminokonzentration herabsetzt und eventuell noch zusätzliche inhibierende Effekte auf die Reaktion haben könnte. Sinnvollerweise sollte diese Reaktion also nur für eine beschränkte Anzahl von Batch-Läufen angewendet werden.

Ein über lange Zeit ablaufendes kontinuierliches Verfahren ist wegen der Instabilität des Amins nicht ratsam.

**Patentansprüche**

**1.** Clostripain mit einer Enzymaktivität von mindestens 80 U/mg, erhältlich aus Clostridium histolyticum DSM 627 durch
- Fermentation von Clostridium histolyticum DSM 627
- Herstellung eines Kulturfiltrats
- Alkoholfällung des Clostripains aus dem Kulturfiltrat
- Adsorption des alkoholgefällten Clostripains an Reaktivfarbstoff.

2. Verfahren zur Herstellung von Clostripain, dadurch gekennzeichnet, daß Clostripain mit einer Enzymaktivität von mindestens 80 U/mg durch Fermentation von Clostridium histolyticum DSM 627 hergestellt wird.

3. Verfahren nach Anspruch 2, dadurch gekennzeichnet, daß Clostripain aus der Kulturbrühe von Clostridium histolyticum DSM 627 durch Filtration, Ultrafiltration, Ammoniumsulfatfällung, Alkoholfällung, hydrophobe Chromatographie oder Affinitätschromatographie hergestellt wird.

4. Verfahren nach Anspruch 2, dadurch gekennzeichnet, daß Clostripain aus der Kulturbrühe von Clostridium histolyticum DSM 627 durch Alkoholfällung des Clostripains aus dem Kulturfiltrat und anschließender Adsorption des alkoholgefällten Enzyms an Reaktivfarbstoff gewonnen wird.

5. Verfahren nach Anspruch 4, dadurch gekennzeichnet, daß für die Alkoholfällung des Clostripains Methanol verwendet wird.

6. Verwendung des Clostripains nach Anspruch 1 oder des nach einem oder mehreren der Ansprüche 2 bis 5 erhältlichen Clostripains zur Verknüpfung von aminoterminal geschütztem Arginin oder von aminoterminal geschützten Peptiden mit carboxyterminalem Arginin (Acyldonor) mit einer Aminosäure oder einem Peptid mit Aminodonor-Funktion, welche jeweils eine Carboxylschutzgruppe, ein Carbonsäureamid oder -hydrazid besitzen.

7. Verwendung des Clostripains nach Anspruch 1 oder des nach einem oder mehreren der Ansprüche 2 bis 5 erhältlichen Clostripains zur Verknüpfung einer Aminosäure oder eines Peptids mit einer nichtproteinogenen Aminosäure, welche Amindonor-Funktion hat, oder mit einem Peptid, welches eine derartige Aminosäure besitzt.

8. Verwendung des Clostripains nach Anspruch 1 oder des nach einem oder mehreren der Ansprüche 2 bis 5 erhältlichen Clostripains, dadurch gekennzeichnet, daß Clostripain in immobilisierter Form eingesetzt wird.

9. Verwendung des Clostripains nach Anspruch 1 oder des nach einem oder mehreren der Ansprüche 2 bis 5 erhältlichen Clostripains, dadurch gekennzeichnet, daß das Reaktions- und Extraktionsmedium der Peptidverknüpfung eine Mischphasen-Lösung ist.